# EUROPEAN PATENT APPLICATION

(11) **EP 0 931 559 A2**
(43) Date of publication of application: **28.07.1999**
(21) Application number: 99300123.9
(22) Date of filing: 07.01.1999
(51) Int. Cl.: A61M 25/00

(54) **Epidural catheters**

(30) Priority: 23.01.1998 GB 9801325
(71) Applicant: Smiths Industries Public Limited Company, London, NW11 8DS (GB)
(72) Inventor: Batchelor, Kester Julian, Burnham-on-Sea, Somerset TA8 2RY (GB); Nash, John Edward, Hythe, Kent CT21 6QU (GB)
(74) Representative: Flint, Jonathan McNeill

(57) **Abstract**

Parallel slits 7 in the wall of the an epidural catheter form four strips 8 which are attached at both ends but which can deflect outwardly midway along their length to anchor the catheter in the epidural space. A tether 5 fixed to the patient end 3 of the catheter can be pulled at its machine end 6 to deflect the strips 8 outwardly. Alternatively, the patient end is split into two strips 18 fixed at their rear end only and having a J-shape memory so that they expand when pushed out of an epidural needle.

## Description

This invention relates to epidural catheters of the kind having a machine end and a patient end that can be inserted into the epidural space through a needle.

Epidural catheters are used to administer anaesthetic fluid to the epidural space in order to relieve pain in the lower part of the body. The patient end tip of the catheter is introduced to the epidural space through a needle, which is subsequently removed over the catheter. The catheter is left in place and taped in position where it emerges from the skin. One problem is that there is a risk that the catheter can be pulled out of the epidural space as the patient moves, because of relative sliding movement between the different tissue layers through which the catheter passes. This can prevent the anaesthetic being administered. In order to reduce this risk, the clinician may insert an extra length of catheter into the epidural space. This extra length of catheter, however, allows the tip to be displaced away from the centre line, thereby increasing the risk of a unilateral block. Also, where an excessive length of catheter is inserted in the epidural space, there is a risk of entanglement of the catheter. In US 5591132 there is described an epidural catheter that forms a curl at its patient end.

It is an object of the present invention to provide an improved epidural catheter.

According to the present invention there is provided an epidural catheter of the above-specified kind, characterised in that a region towards the patient end has at least two deflectable members that can deflect away from one another when outside the needle in the epidural space such as to anchor the patient end in the epidural space.

The deflectable members may possess a memory such that they deflect away from one another when unconfined. The deflectable members may be formed from a part of the wall of the catheter and may be formed by dividing the wall of the catheter in the patient end region with a plurality of parallel slits into a plurality of deflectable strips.

In one form, the deflectable members may be attached with the wall of the catheter at both ends, the deflectable members deflecting outwardly midway along their length. The catheter may include a manually-engageable member extending along the catheter from the patient end region to the machine end, the manually-engageable member being movable along the catheter such as to displace the deflectable members. The manually-engageable is preferably attached with the patient end of the catheter forwardly of the deflectable members. The patient end tip of the catheter may be closed, the patient end of the catheter opening through the wall of the catheter in the patient end region.

Alternatively, the deflectable members may be attached with the wall of the catheter at their rear ends only such that the forward ends of the deflectable members are deflectable outwardly. The deflectable members may be formed by splitting the catheter in half into two deflectable members.

Two forms of epidural catheter according to the present invention, will now be described, by way of example, with reference to the accompanying drawings, in which:
- Figure 1: is a perspective view of the first form of catheter, in an unexpanded state;
- Figure 2: is a perspective view of the catheter of Figure 1, in an expanded state;
- Figure 3: is an enlarged perspective view of the patient end of the catheter of Figures 1 and 2 in an expanded state;
- Figure 4: is a perspective view of a second form of catheter;
- Figure 5: is an enlarged perspective view of the patient end of the catheter of Figure 4; and
- Figure 6: is a perspective view of the catheter of Figures 4 and 5, showing the catheter prior to use.

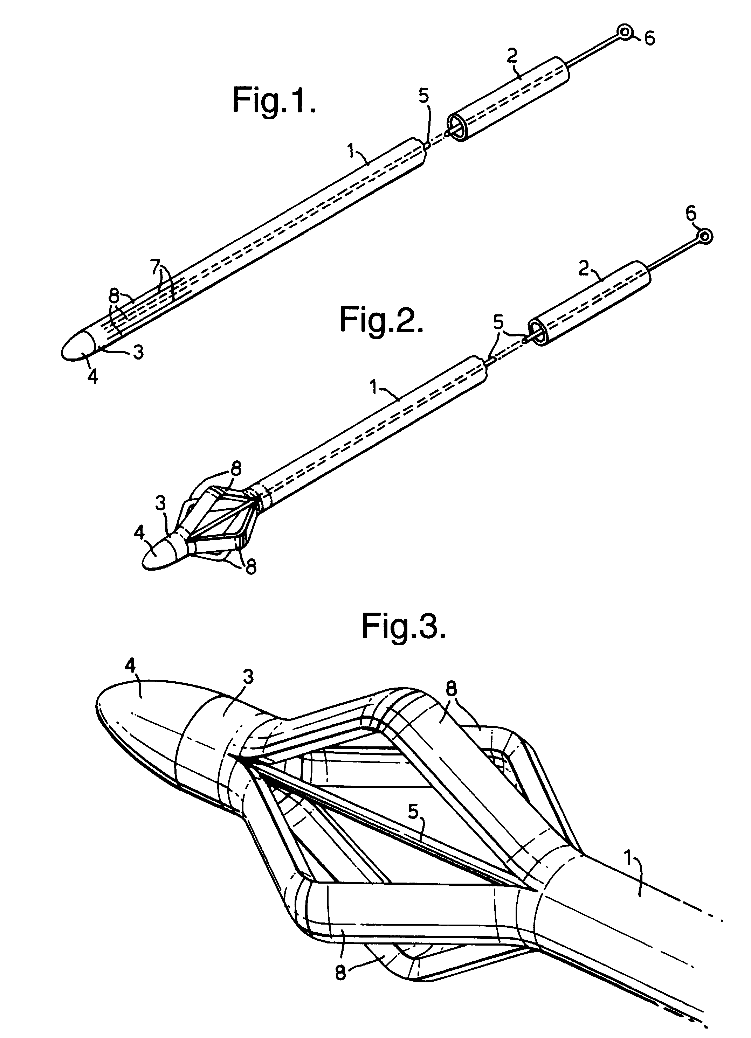

With reference first to Figures 1 to 3, the catheter has a nylon tubular shaft 1 with a circular section and a diameter of about 1mm. The length of the catheter is typically about 920mm. The rear, machine end 2 of the catheter is plain so that the needle used to introduce the catheter can be pulled off over the machine end of the catheter. The patient end 3 of the catheter is closed by a bullet-shape tip 4, which is attached to the forward end of a flexible tether 5 extending along the inside of the shaft 1. The rear end of the tether 5 emerges from the rear end 2 of the catheter and is formed with a small loop 6, which is large enough to prevent it entering the bore of the catheter but is small enough to allow it to pass through the bore of the needle used to introduce the catheter. The cross-sectional dimensions of the tether 5 are small enough to allow passage of fluid along the catheter. About 5mm rearwardly of its patient end 3, the wall of the catheter is cut by four parallel slits 7 to divide a region of the catheter into four longitudinally-extending strip members 8 about 15mm long. The strips are attached at both ends with the shaft 1. In their natural state, the strip members 8 lie flat, forming a continuation of the cylindrical surface of the remainder of the shaft 1, as shown in Figure 1. It is in this state that the catheter is introduced through a hollow needle into the epidural space, in the usual way.

After removing the needle, the loop 6 is gripped and the tether 5 is pulled, so as to pull the tip 4 rearwardly by about 10mm. As this happens, the strip members 8 deflect outwardly away from one another, in the manner shown in Figures 2 and 3, so that the lateral dimensions of the catheter in this region are substantially increased. The expanded cross section of the catheter in this region is sufficient to prevent it being pulled through the path made through the tissue by the needle. This, therefore, anchors the patient end of the catheter in the epidural space until the tether 5 is released. The machine end 2 of the catheter is attached to a modified epidural catheter connector (not shown), which acts to hold the loop 6 in its retracted position and allows anaesthetic liquid to be injected along the catheter. The anaesthetic liquid flows out of the patient end 3 of the catheter through the expanded slits 7 between the strips 8. The catheter could be pulled rearwardly by the clinician, following deflection of the strips 8, until the strips contact the wall of the epidural space. This ensures that the anaesthetic fluid is administered directly at the midline. When the catheter needs to be removed, the loop 6 is released, allowing the resilience of the deflectable strip members 8 to restore them to their original flat state.

With this arrangement, there is a reduced the risk of the patient end of the catheter being pulled out of the epidural space without the need to increase the length of catheter inserted in the epidural space, thereby avoiding the consequent increase in the risk of unilateral block.

It will be appreciated that the natural state of the deflectable members 8 could be such that they are in a laterally expanded state, in which case, the tether would be sufficiently stiff that, when pushed forwardly, it pushes the patient end forwardly and thereby retracts the deflectable members inwardly sufficient for insertion and removal.

Instead of forming the deflectable strips from the wall of the shaft of the catheter itself, they could be formed from a slit sleeve attached over a conventional epidural catheter.

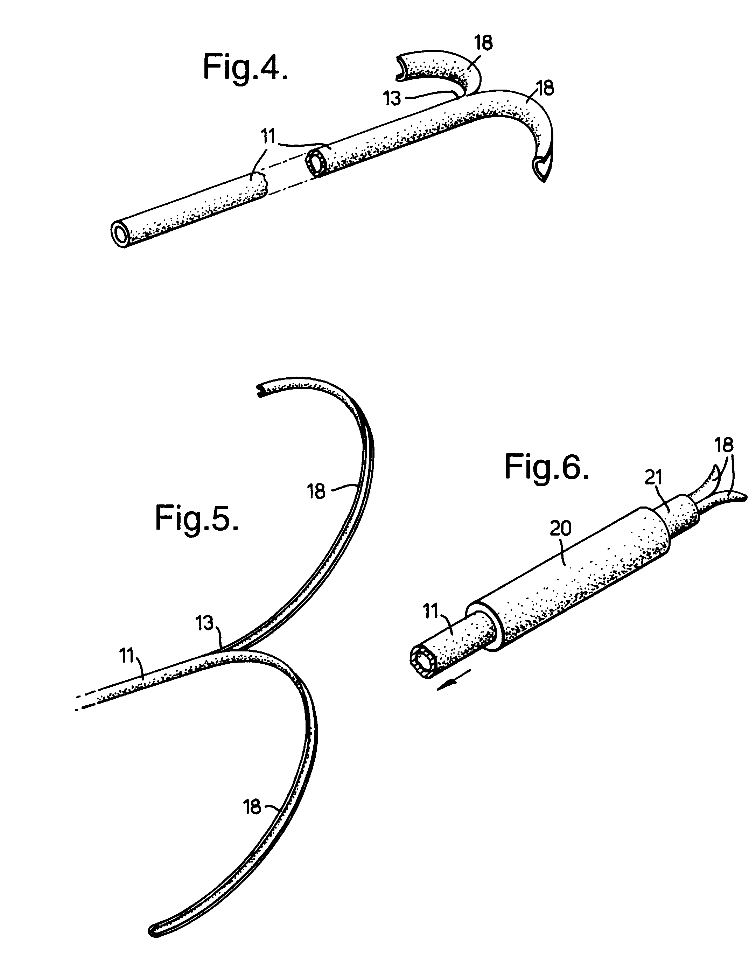

There are various other ways in which an epidural catheter could be provided with deflectable members to anchor the patient end of the catheter in the epidural space. For example, as shown in Figures 4 to 6, the shaft 11 of the catheter could simply be split longitudinally at its patient end 13 into two arms 18. The arms 18 are attached with the shaft at their machine end and are unattached at their patient end. The arms 18 have a plastic memory (such as provided by suitable heat treatment) such that they tend to assume a J-shape extending laterally away from the axis of the main part of the catheter. The catheter is introduced in the manner shown in Figure 6. The catheter is pulled rearwardly into a needle introducer 20 in the form of a short sleeve having a reduced external diameter at its forward end 21. This brings the arms 18 together. The forward end 21 of the introducer 20 is then inserted into the hub of the epidural needle (not shown) so that the bore of the introducer aligns with the bore of the needle. The catheter is then pushed forwardly out of the introducer and along the bore of the needle, with the arms 18 being held together by the confinement of the needle. When the catheter emerges from the patient end of the needle, in the epidural space, the arms 18 deflect laterally outwardly, by their memory. The needle is then removed over the rear end of the catheter, leaving the catheter in position. The deflected arms 18 anchor the patient end of the catheter in the epidural space. To remove the catheter, it is simply pulled out with sufficient force to close the two arms 18 sufficiently for them to pass through the passage through the tissue made by the epidural needle. This force is greater than that normally experienced as a result of movement of patient tissue. Because the patient end of this form of catheter has relatively large lateral dimensions, there is a low risk of dural tap, that is, of the patient end of the catheter puncturing the dura and entering the spinal cavity.

## Claims

1. An epidural catheter having a machine end (2) and a patient end (3, 13) that can be inserted into the epidural space through a needle, characterised in that a region towards the patient end has at least two deflectable members (8, 18) that can deflect away from one another when outside the needle in the epidural space such as to anchor the patient end (3, 13) in the epidural space.

2. An epidural catheter according to Claim 1, characterised in that the deflectable members (8, 18) possess a memory such that they deflect away from one another when unconfined.

3. An epidural catheter according to Claim 1 or 2, characterised in that the deflectable members (8, 18) are formed from a part of the wall of the catheter.

4. An epidural catheter according to Claim 3, characterised in that the deflectable members (8, 18) are formed by dividing the wall of the catheter in the patient end region with a plurality of parallel slits (7) into a plurality of deflectable strips (8, 18).

5. An epidural catheter according to Claim 3, characterised in that the deflectable members (8) are attached with the wall of the catheter at both ends, and that the deflectable members (8) deflect outwardly midway along their length.

6. An epidural catheter according to any one of the preceding claims, characterised in that the catheter includes a manually-engageable member (5) extending along the catheter from the patient end region to the machine end (2), and that the manually-engageable member (5) is movable along the catheter such as to displace the deflectable members (8).

7. An epidural catheter according to Claim 6, characterised in that the manually-engageable member (5) is attached with the patient end (3) of the catheter forwardly of the deflectable members (8).

8. An epidural catheter according to any one of the preceding claims, characterised in that the patient end tip (4) of the catheter is closed and the patient end of the catheter opens through the wall of the catheter in the patient end region.

9. An epidural catheter according to any one of Claims 1 to 4, characterised in that the deflectable members (18) are attached with the wall of the catheter at their rear end only such that the forward end of the deflectable members are deflectable outwardly.

10. An epidural catheter according to Claim 9, characterised in that the deflectable members (18) are formed by splitting the catheter in half into two deflectable members.
